# EUROPEAN PATENT APPLICATION

(11) **EP 0 875 571 A2**
(43) Date of publication of application: **04.11.1998**
(21) Application number: 98303237.6
(22) Date of filing: 27.04.1998
(51) Int. Cl.: C12N 15/12, C12N 5/10, C07K 14/47, C07K 16/18, C12Q 1/68, G01N 33/566

(54) **CRFG-1, target and marker for chronic renal failure**

(30) Priority: 30.04.1997 US 45203 P; 10.12.1997 US 988028
(71) Applicant: SMITHKLINE BEECHAM CORPORATION, Philadelphia Pennsylvania 19103 (US)
(72) Inventor: Laping, Nicholas, SmithKline Beecham Pharm., King of Prussia, Pennsylvania 19406 (US); Olson, Barbara, SmithKline Beecham Pharm., King of Prussia, Pennsylvania 19406 (US); Zhu, Yuan, SmithKline Beecham Pharm., King of Prussia, Pennsylvania 19406 (US)
(74) Representative: Crump, Julian Richard John

(57) **Abstract**

Chronic renal failure gene-1 polypeptides and polynucleotides and methods for producing such polypeptides by recombinant techniques are disclosed. Also disclosed are methods for utilizing chronic renal failure gene-1 polypeptides and polynucleotides in the design of protocols for the treatment of chronic renal disease, renal ischemia, diabetic nephropathy, acute renal failure, and Alzheimer's disease, among others, and diagnostic assays for such conditions.

### Annex to the description

SEQUENCE LISTING

(1) GENERAL INFORMATION

(i) APPLICANT: SmithKline Beecham Corporation

(ii) TITLE OF THE INVENTION: CRFG-1, a target and marker
for chronic renal failure

(iii) NUMBER OF SEQUENCES: 6

(iv) CORRESPONDENCE ADDRESS:
(A) ADDRESSEE: SmithKline Beecham, Corporate Intellectual
Property
(B) STREET: Two New Horizons Court
(C) CITY: Brentford
(D) STATE: Middlesex
(E) COUNTRY: United Kingdom
(F) ZIP: TW8 9EP

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER: TO BE ASSIGNED
(B) FILING DATE: 10-DEC-1997
(C) CLASSIFICATION: UNKNOWN

(vii) PRIOR APPLICATION DATA:
(A) APPLICATION NUMBER: 60/045,203
(B) FILING DATE: 30-APR-1997

(viii) ATTORNEY/AGENT INFORMATION:
(A) NAME: CONNELL, Anthony Christopher
(B) REGISTRATION NUMBER: 5630
(C) REFERENCE/DOCKET NUMBER: GH70009

(ix) TELECOMMUNICATION INFORMATION:
(A) TELEPHONE: +44 1279 644 395

(B) TELEFAX: +44 181 975 6294
(C) TELEX:

(2) INFORMATION FOR SEQ ID NO:1:

(i) SEQUENCE CHARACTERISTICS:
(A) LENGTH: 2682 base pairs
(B) TYPE: nucleic a[?]id
(C) STRANDEDNESS: single
(D) TOPOLOGY: linear

(ii) MOLECULE TYPE: cDNA

(xi) SEQUENCE DESCRIPTION: SEQ ID NO:1:

TTAAAAATCA CATTATTGGC CAGGTGCAGT GGCTCATGCC TGTAATCCCA GCCCTTTGGG 60
AGGCCGAGGC AGGTGGATCA CAAGGTCAGG AGATTGAGAC CATCCTGGTT AACACGGTGA 120
AACCCCGTGT CTACTAAAAA TACAAAAAAT TAGCTGGGTG TGGTGGCGGG CGCCTGTGGT 180
CCCAGCTACT CGGGAGGCTG AAGCAGGACA ATGGCGTGAA CCCAGGAAGT GGAGCTTGCA 240
GTGAGCCAAG ATTGCGCCAT TGCGTCTTCC CAACAGAACA AGACTCCATC TCAAAAAATA 300
AATAAATAAA TAAGCGGGCA CATTACAACT TCAAGAAAAT TACGGTGGTG CCGTCCGCCA 360
AGGACTTCAT AGACCTCACG TTGTCG[?]AGA CTCAACGAAA GACTCCAACC GTTATTCATA 420
AACATTACCA AATACATCGC ATTAGACATT TTTACATGAG AAAAGTCAAA TTTACTCAAC 480
AGAATTACCA TGATAGACTT TCACAAATTC TAACAGATTT CCCCAAATTG GATGATATTC 540
ATCCGTTCTA TGCTGATTTG ATGAATATTC TCTACGACAA GGATCATTAC AAGTTGGCTC 600
TGGGGCAAAT AAATATTGCC AAAAATTTAG TGGACAATGT TGCTAAAGAT TATGTGCGAC 660
TGATGAAGTA TGGCGACTCT CTCTACCGCT GCAAACAGCT GAAGCGTGCG GCCCTGGGAC 720
GGATGTGCAC AGTGATCAAG AGGCAGAAGC AGAGTTTGGA GTATTTGGAG CAAGTGCGTC 780
AGCATTTATC CCGTTTGCCA ACCATTGATC CGAATACCAG GACCCTGCTT TTGTGTGGGT 840
ACCCAAATGT TGGGAAGTCC AGCTTCATCA ACAAGGTGAC GAGAGCAGAC GTGGATGTCC 900
AGCCCTATGC GTTCACAACC AAGTCTCTGT TTGTTGGGCA CATGGATTAT AAGTATCTAC 960
GCTGGCAGGT TGTAGACACT CCTGGGATCC TGGACCACCC TCTGGAGGAT AGGAACACCA 1020
TCGAGATGCA GGCCATCACT GCCCTGGCCC ACCTCCGTGC TGCGGTCCTG TATGTGATGG 1080
ATTTGTCTGA GCAGTGTGGG CATGGGCTGA GGGAACAGCT AGAACTCTTC CAGAACATCA 1140
GACCTCTCTT CATCAACAAG CCTCTCATAG TTGTAGCCAA CAAATGTGAT GTGAAGAGAA 1200
TAGCTGAACT TTCTGAAGAT GATCAG[?]AAA TATTTACAGA TTTGCAGTCT GAAGGATTCC 1260
CTGTAATAGA AACCAGCACC CTGACTGAGG GAGGTGTTAT TAAAGTTAAA ACAGAGGCTA 1320
GCGATAGGCT TTTGGCTCAT CGAGTGGAAA CCAAGATGAA GGGAAATAAA GTGAATGAGC 1380
TGCTGAATAG ACTGCACCTG GCTATCCCAA CCAGGAGGGA CGATAAGGAG AGGCCCCCTT 1440
TCATCCCTGA AGGAGTGGTG GCTCGCAGGA AGAGGATGGA AACTGAGGAG TCCAGGAAGA 1500
AGAGGGAACG AGATCTTGAG CTGGAAATGG GAGATGATTA TATTTTGGAT CTTCAGAAGT 1560
ACTGGGATTT AATGAATTTG TCTGAAAAAC ATGATAAGAT ACCAGAAATC TGGGAAGGCC 1620

ATAATATAGC TGATTATATT GATCCAGCCA TCATGAAGAA ATTGGAAGAA TTAGAAAAAG 1680
AAGAAGAGCT GAGAACAGCT GCTGGAGAGT ATGACAGTGT ATCTGAGAGT GAAGACGAAG 1740
AGATGCTGGA AATCCGACAG CTGGCAAAGC AAATTCGAGA GAAAAAGAAG TTGAAAATTC 1800
TGGAGTCCAA AGAAAAGAAT ACACAGGGAC CCAGGATGCC GCGAACTGCT AAGAAGGTTC 1860
AGAGGACAGT TTTGGAGAAG GAGATGCGTA GTCTTGGTGT TGACATGGAC GATAAAGACG 1920
ATGCCCATTA CGCAGTCCAG GCAAGAAGAT CCCGGAGCAT CACTAGGAAA AGAAAGCGGG 1980
AAGACTCTGC TCCCCCGTCC TCTGTGGCCC GGAGTGGGAG TTGCTCTCGA ACTCCACGTG 2040
ACGTTTCTGG TCTTAGGGAT GTCAAGATGG TGAAGAAAGC CAAGACTATG ATGAAGAATG 2100
CTCAGAAGAA GATGAATCGG TTGGGGAAGA AAGGGGAGGC GGATAGACAC GTGTTTGATA 2160
TGAAGCCCAA GCACTTGCTG TCTGGGAAGA GGAAAGCTGG TAAAAAGGAC AGGAGATAGT 2220
ATCCGTTTGG TTGGCGTGGC TTCGCTAGAG TGTTGCTGTT TATTTCCTGT TTTGGCACAG 2280
TATGGTTTCA TGAAATTGGA GCTCTGTATA AACTGAAAAA GACAAAATAA GTAAAGCACT 2340
TGTTGCTTTG CTGAAAACTA TGGTTAACCC TATATAGGTG TGGGAAATTT TTGTCACTGC 2400
ATAATATTAC AAATATTTTG AGTAGACAGT GTTTCCACAT TTAATGGAGT ATCAGTTGCT 2460
TCAGATTTTC AGAACTGGGA AGATTTACTG GTGTAACTGG GTTGTTTTTG ATGGAGAAAA 2520
ACCTTATTTT CTTTTGTAAG AGCTGGGAGC AAACACGTTT ATGAGTGTGT CGGAATCCCG 2580
TGCTTAAAAT ACGCTCTTAA ATTATTTTCT AGTCTTATTT CACAATGTCT CATTGTAGTC 2640
TGTCTTCAAC TATTTTATCC AAAATANACC TCCAGAAGAA AG 2682

(2) INFORMATION FOR SEQ ID NO:2:

(i) SEQUENCE CHARACTERISTICS:
(A) LENGTH: 587 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:2:

Met Arg Lys Val Lys Phe Thr Gln Gln Asn Tyr His Asp Arg Leu Ser
1               5                   10                  15
Gln Ile Leu Thr Asp Phe Pro Lys Leu Asp Asp Ile His Pro Phe Tyr
            20                  25                  30
Ala Asp Leu Met Asn Ile Leu Tyr Asp Lys Asp His Tyr Lys Leu Ala
        35                  40                  45
Leu Gly Gln Ile Asn Ile Ala Lys Asn Leu Val Asp Asn Val Ala Lys
    50                  55                  60
Asp Tyr Val Arg Leu Met Lys Tyr Gly Asp Ser Leu Tyr Arg Cys Lys
65                  70                  75                  80
Gln Leu Lys Arg Ala Ala Leu Gly Arg Met Cys Thr Val Ile Lys Arg
                85                  90                  95

Gln Lys Gln Ser Leu Glu Tyr Leu Glu Gln Val Arg Gln His Leu Ser
            100                 105                 110
Arg Leu Pro Thr Ile Asp Pro Asn Thr Arg Thr Leu Leu Leu Cys Gly
        115                 120                 125
Tyr Pro Asn Val Gly Lys Ser Ser Phe Ile Asn Lys Val Thr Arg Ala
    130                 135                 140
Asp Val Asp Val Gln Pro Tyr Ala Phe Thr Thr Lys Ser Leu Phe Val
145                 150                 155                 160
Gly His Met Asp Tyr Lys Tyr Leu Arg Trp Gln Val Val Asp Thr Pro
                165                 170                 175
Gly Ile Leu Asp His Pro Leu Glu Asp Arg Asn Thr Ile Glu Met Gln
            180                 185                 190
Ala Ile Thr Ala Leu Ala His Leu Arg Ala Ala Val Leu Tyr Val Met
        195                 200                 205
Asp Leu Ser Glu Gln Cys Gly His Gly Leu Arg Glu Gln Leu Glu Leu
    210                 215                 220
Phe Gln Asn Ile Arg Pro Leu Phe Ile Asn Lys Pro Leu Ile Val Val
225                 230                 235                 240
Ala Asn Lys Cys Asp Val Lys Arg Ile Ala Glu Leu Ser Glu Asp Asp
                245                 250                 255
Gln Lys Ile Phe Thr Asp Leu Gln Ser Glu Gly Phe Pro Val Ile Glu
            260                 265                 270
Thr Ser Thr Leu Thr Glu Gly Gly Val Ile Lys Val Lys Thr Glu Ala
        275                 280                 285
Ser Asp Arg Leu Leu Ala His Arg Val Glu Thr Lys Met Lys Gly Asn
    290                 295                 300
Lys Val Asn Glu Leu Leu Asn Arg Leu His Leu Ala Ile Pro Thr Arg
305                 310                 315                 320
Arg Asp Asp Lys Glu Arg Pro Pro Phe Ile Pro Glu Gly Val Val Ala
                325                 330                 335
Arg Arg Lys Arg Met Glu Thr Glu Glu Ser Arg Lys Lys Arg Glu Arg
            340                 345                 350
Asp Leu Glu Leu Glu Met Gly Asp Asp Tyr Ile Leu Asp Leu Gln Lys
        355                 360                 365
Tyr Trp Asp Leu Met Asn Leu Ser Glu Lys His Asp Lys Ile Pro Glu
    370                 375                 380
Ile Trp Glu Gly His Asn Ile Ala Asp Tyr Ile Asp Pro Ala Ile Met
385                 390                 395                 400
Lys Lys Leu Glu Glu Leu Glu Lys Glu Glu Glu Leu Arg Thr Ala Ala
                405                 410                 415
Gly Glu Tyr Asp Ser Val Ser Glu Ser Glu Asp Glu Glu Met Leu Glu
            420                 425                 430
Ile Arg Gln Leu Ala Lys Gln Ile Arg Glu Lys Lys Lys Leu Lys Ile

        435                 440                 445
Leu Glu Ser Lys Glu Lys Asn Thr Gln Gly Pro Arg Met Pro Arg Thr
    450                 455                 460
Ala Lys Lys Val Gln Arg Thr Val Leu Glu Lys Glu Met Arg Ser Leu
465                 470                 475                 480
Gly Val Asp Met Asp Asp Lys Asp Asp Ala His Tyr Ala Val Gln Ala
                485                 490                 495
Arg Arg Ser Arg Ser Ile Thr Arg Lys Arg Lys Arg Glu Asp Ser Ala
            500                 505                 510
Pro Pro Ser Ser Val Ala Arg Ser Gly Ser Cys Ser Arg Thr Pro Arg
        515                 520                 525
Asp Val Ser Gly Leu Arg Asp Val Lys Met Val Lys Lys Ala Lys Thr
    530                 535                 540
Met Met Lys Asn Ala Gln Lys Lys Met Asn Arg Leu Gly Lys Lys Gly
545                 550                 555                 560
Glu Ala Asp Arg His Val Phe Asp Met Lys Pro Lys His Leu Leu Ser
                565                 570                 575
Gly Lys Arg Lys Ala Gly Lys Lys Asp Arg Arg
            580                 585

(2) INFORMATION FOR SEQ ID NO:3:

(i) SEQUENCE CHARACTERISTICS:
(A) LENGTH: 716 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:3:

GACTCTGCTC CCCCGTCCTC TGTGGCCCGG AGTGGGAGTT GCTCTCGAAC TCCACGTGAC 60
GTTTCTGGTC TTAGGGATGT CAAGATGGTG AAGAAAGCCA AGACTATGAT GAAGAATGCT 120
CAGAAGAAGA TGAATCGGTT GGGGAAGAAA GGGGAGGCGG ATATACACTT GTTTGATATG 180
AAGCCCAAGC ACTTGCTGTC TGGGAAGAGG AAAGCTGGTA AAAAGGACAG GAGATAGTAT 240
CCGTTTGGTT GGCGTGGCTT CGCTAGAGTG TTGCTGTTTA TTTCCTGGTT TGGCACAGTA 300
TGGTTTCATG AAATTGGAGC TCTGTATAAA CTGAAAAAGA CAAAATAAGT AAAGCACTTG 360
TTGCTTTGCT GAAAACTATG GTTAACCCTA TATAGGTGTG GGAAATTTTT GTCACTGCAT 420
AATATTACAA ATATTCTGAG TAGACAGTGT TTCCACATTT AATGGAGTAT CAGTTGCTTC 480
AGATTTTCAG AACTGGGAAG ATTTACTGGT GTAACTGGGT TGTTTTTGAT GGAGAAAAAC 540
CTTATTTTCT TTTGTAAGAG CTGGGAGCAA ACACGTTTAT GAGTGTGTCG GAATCCCGTG 600
CTTAAAATAC GCTCTTAAAT TATTTTCTAG TCCTTATTTT ACAATGTCTC ATTGTAGTCT 660

GTCTTCAACT ATTTTATCCA AAATAAACCT CCAGAAGGAA AAAAAAAAAA AAAAAA 716

(2) INFORMATION FOR SEQ ID NO:4:

(i) SEQUENCE CHARACTERISTICS:
(A) LENGTH: 104 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:4:

His Glu Asp Lys Asp Asp Ala His Tyr Ala Val Gln Ala Arg Arg Ser
1               5                   10                  15
Arg Ser Ile Thr Arg Lys Arg Lys Arg Glu Asp Ser Ala Pro Pro Ser
            20                  25                  30
Ser Val Ala Arg Ser Gly Ser Cys Ser Arg Thr Pro Arg Asp Val Ser
        35                  40                  45
Gly Leu Arg Asp Val Lys Met Val Lys Lys Ala Lys Thr Met Met Lys
    50                  55                  60
Asn Ala Gln Lys Lys Met Asn Arg Leu Gly Lys Lys Gly Glu Ala Asp
65                  70                  75                  80
Ile His Leu Phe Asp Met Lys Pro Lys His Leu Leu Ser Gly Lys Arg
                85                  90                  95
Lys Ala Gly Lys Lys Asp Arg Arg
            100

(2) INFORMATION FOR SEQ ID NO:5:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:5:

ACCACACATC TGA 13

(2) INFORMATION FOR SEQ ID NO:6:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:6:

TGTTGGGAAC AAG 13

## Description

This application claims the benefit of U.S. Provisional Application No. 60/045,203, filed April 30, 1997.

### FIELD OF INVENTION

This invention relates to newly identified polynucleotides, polypeptides encoded by them and to the use of such polynucleotides and polypeptides, and to their production. More particularly, the polynucleotides and polypeptides of the present invention relate to GTP binding protein family, hereinafter referred to as chronic renal failure gene-1. The invention also relates to inhibiting or activating the action of such polynucleotides and polypeptides.

### BACKGROUND OF THE INVENTION

Two animal model systems have been studied to provide targets for intervention in chronic renal failure. CRFG-1 is a novel gene identified by differential display PCR to be down regulated in three animal models of chronic renal failure, the obese Zucker rat and the 5/6 nephrectomized rat. In addition, aged Fisher 344 rats (24 mo old), which have enlarged kidneys and reduced renal function, also have decreased expression of CRFG-1. Loss of expression of this gene in renal failure may indicate an important role in normal functioning of the kidney.

In 5-month old obese Zucker rats that have developed chronic renal failure, CRFG-1 mRNA is decreased to less than 1/3 of levels seen in lean and healthy age-matched controls. Because there is no correlation of proteinuria with CRFG-1 expression, a decrease in CRFG-1 is an early marker of renal impairment before standard clinical indicators.

While in the rat, CRFG-1 has 2 mRNA sizes of 2.5 and 1.5 kb, in the human only one molecular weight species is identified at 2.7 kb. Human CRFG-1 was mapped to human chromosome 10p15.2-15. This region of chromosome 10 is linked to 171840 PHOSPHOFRUCTOKINASE, PLATELET TYPE, 600449 DIHYDRODIOL DEHYDROGENASE, TYPE I; 601070 INTERLEUKIN-15 RECEPTOR, ALPHA; 600448 PROTEIN KINASE C, THETA FORM; 147270 INTER-ALPHA-TRYPSIN INHIBITOR, HEAVY CHAIN-1; 176870 PROTEIN HC; 137800 GLIOMA OF BRAIN, 300007 INTERLEUKIN-9 RECEPTOR; and 147680 INTERLEUKIN-2.

Normal mRNA expression on a multiple tissue northern blot identifies expression in skeletal muscle > testes > pancreas > heart > thymus > placenta > kidney > leukocytes > spleen > ovary > colon > brain > prostate.

The sequence of CRFG-1 is similar to uncharacterized putative GTP binding proteins of yeast (YPL093w), Halobacterium cutirubrum and GTP1/OBG family of GTP binding proteins from Methanobacterium thermoautotrophicum. GTP binding proteins play important roles in intracellular transport, protein targeting and vesicle fusion.

This indicates that the GTP binding protein family has an established, proven history as therapeutic targets. Clearly there is a need for identification and characterization of further members of GTP binding protein family which can play a role in preventing, ameliorating or correcting dysfunctions or diseases, including, but not limited to, chronic renal disease, renal ischemia, diabetic nephropathy, acute renal failure, and Alzheimer's disease.

### SUMMARY OF THE INVENTION

In one aspect, the invention relates to chronic renal failure gene-1 polypeptides and recombinant materials and methods for their production. Another aspect of the invention relates to methods for using such chronic renal failure gene-1 polypeptides and polynucleotides. Such uses include the treatment of chronic renal disease, renal ischemia, diabetic nephropathy, acute renal failure, and Alzheimer's disease, among others. In still another aspect, the invention relates to methods to identify agonists and antagonists using the materials provided by the invention, and treating conditions associated with chronic renal failure gene-1 imbalance with the identified compounds. Yet another aspect of the invention relates to diagnostic assays for detecting diseases associated with inappropriate chronic renal failure gene-1 activity or levels.

### DESCRIPTION OF THE INVENTION

### Definitions

The following definitions are provided to facilitate understanding of certain terms used frequently herein.

"Chronic renal failure gene-1" refers, among others, generally to a polypeptide having the amino acid sequence set forth in SEQ ID NO:2 or an allelic variant thereof.

"Chronic renal failure gene-1 activity or chronic renal failure gene-1 polypeptide activity" or "biological activity of the chronic renal failure gene-1 or chronic renal failure gene-1 polypeptide" refers to the metabolic or physiologic function of said chronic renal failure gene-1 including similar activities or improved activities or these activities with decreased undesirable side-effects. Also included are antigenic and immunogenic activities of said chronic renal failure gene-1.

"Chronic renal failure gene-1 gene" refers to a polynucleotide having the nucleotide sequence set forth in SEQ ID NO: 1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al.,* "Analysis for protein modifications and nonprotein cofactors", *Meth Enzymol* (1990) 182:626-646 and Rattan *et al.,* "Protein Synthesis: Posttranslational Modifications and Aging", *Ann NY Acad Sci* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per* *se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al., Nucleic Acids Research* (1984) 12(1):387), BLASTP, BLASTN, FASTA (Atschul, S.F. *et al., J Molec Biol* (1990) 215:403).

As an illustration, by a polynucleotide having a nucleotide sequence having at least, for example, 95% "identity" to a reference nucleotide sequence of SEQ ID NO: 1 is intended that the nucleotide sequence of the polynucleotide is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO: 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides of the Invention

In one aspect, the present invention relates to chronic renal failure gene-1 polypeptides (or chronic renal failure gene-1 proteins). The chronic renal failure gene-1 polypeptides include the polypeptide of SEQ ID NOS:2 and 4; as well as polypeptides comprising the amino acid sequence of SEQ ID NO: 2; and polypeptides comprising the amino acid sequence which have at least 80% identity to that of SEQ ID NO:2 over its entire length, and still more preferably at least 90% identity, and even still more preferably at least 95% identity to SEQ ID NO: 2. Furthermore, those with at least 97-99% are highly preferred. Also included within chronic renal failure gene-1 polypeptides are polypeptides having the amino acid sequence which have at least 80% identity to the polypeptide having the amino acid sequence of SEQ ID NO:2 over its entire length, and still more preferably at least 90% identity, and still more preferably at least 95% identity to SEQ ID NO:2. Furthermore, those with at least 97-99% are highly preferred. Preferably chronic renal failure gene-1 polypeptide exhibit at least one biological activity of chronic renal failure gene-1.

The chronic renal failure gene-1 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the chronic renal failure gene-1 polypeptides are also included in the invention. A fragment is a polypeptide having an amino acid sequence that entirely is the same as part, but not all, of the amino acid sequence of the aforementioned chronic renal failure gene-1 polypeptides. As with chronic renal failure gene-1 polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of chronic renal failure gene-1 polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of chronic renal failure gene-1 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate chronic renal failure gene-1 activity, including those with a similar activity or an improved activity, or with a decreased undesirable activity. Also included are those that are antigenic or immunogenic in an animal, especially in a human.

Preferably, all of these polypeptide fragments retain the biological activity of the chronic renal failure gene-1, including antigenic activity. Among the most preferred fragment is that having the amino acid sequence of SEQ ID NO: 4. Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The chronic renal failure gene-1 polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to chronic renal failure gene-1 polynucleotides. Chronic renal failure gene-1 polynucleotides include isolated polynucleotides which encode the chronic renal failure gene-1 polypeptides and fragments, and polynucleotides closely related thereto. More specifically, chronic renal failure gene-1 polynucleotide of the invention include a polynucleotide comprising the nucleotide sequence contained in SEQ ID NO: 1 encoding a chronic renal failure gene-1 polypeptide of SEQ ID NO: 2, and polynucleotides having the particular sequences of SEQ ID NOS: 1 and 3. chronic renal failure gene-1 polynucleotides further include a polynucleotide comprising a nucleotide sequence that has at least 80% identity over its entire length to a nucleotide sequence encoding the chronic renal failure gene-1 polypeptide of SEQ ID NO:2, and a polynucleotide comprising a nucleotide sequence that is at least 80% identical to that of SEQ ID NO: 1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under chronic renal failure gene-1 polynucleotides are a nucleotide sequence which has sufficient identity to a nucleotide sequence contained in SEQ ID NO: 1 to hybridize under conditions useable for amplification or for use as a probe or marker. The invention also provides polynucleotides which are complementary to such chronic renal failure gene-1 polynucleotides.

Chronic renal failure gene-1 of the invention is structurally related to other proteins of the GTP binding protein family, as shown by the results of sequencing the cDNA encoding human chronic renal failure gene-1. The cDNA sequence of SEQ ID NO: 1 contains an open reading frame (nucleotide number 456 to 2216) encoding a polypeptide of 587 amino acids of SEQ ID NO:2. The amino acid sequence of Table 2 (SEQ ID NO:2) has about 45.3% identity (using FASTA) in 587 amino acid residues with Hypothetical protein YPL093w from yeast (H. Bussey et al. Nature 387 (6632 Suppl), 103-105 ,1997). The nucleotide sequence of Table 1 (SEQ ID NO:1) has about 60.2% identity (using FASTA) in 1364 nucleotide residues with Saccharomyces cerevisiae chromosome XVI cosmid 8059/8047 (H. Bussey et al. Nature 387 (6632 Suppl), 103-105 1997). Thus, chronic renal failure gene-1 polypeptides and polynucleotides of the present invention are expected to have, inter alia, similar biological functions/properties to their homologous polypeptides and polynucleotides, and their utility is obvious to anyone skilled in the art.

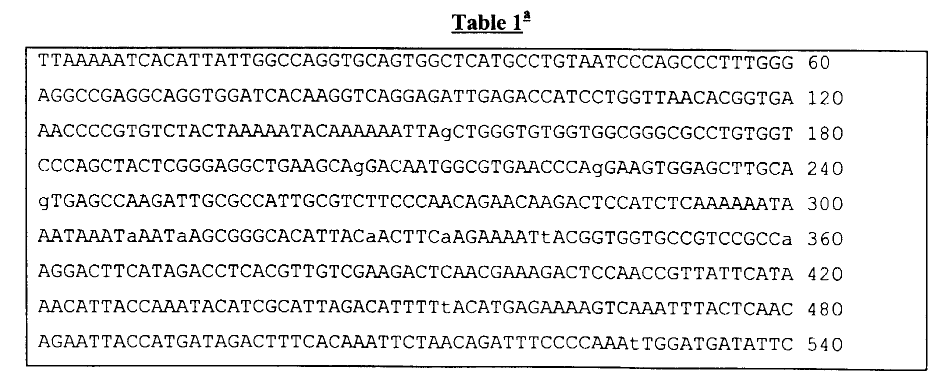

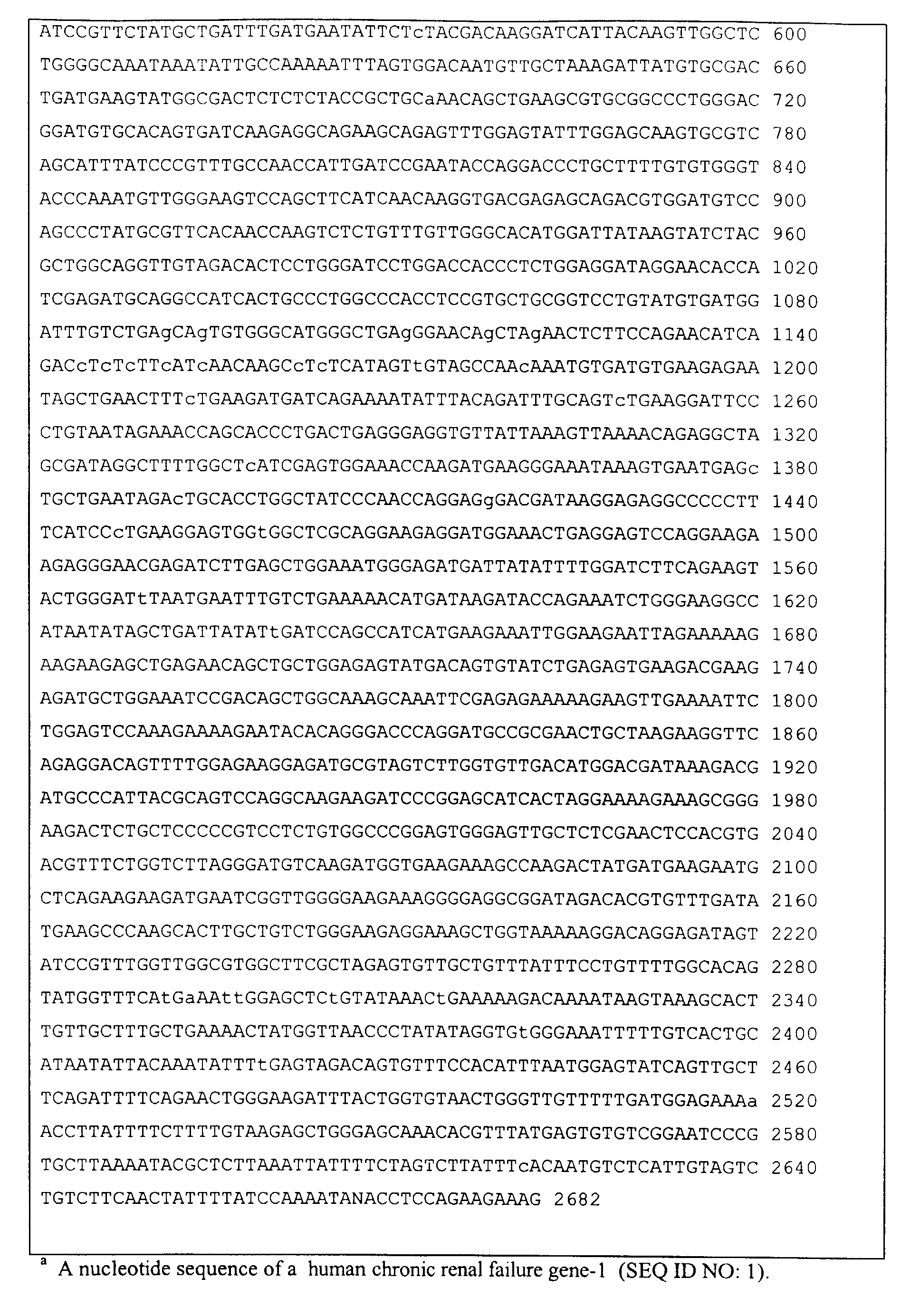

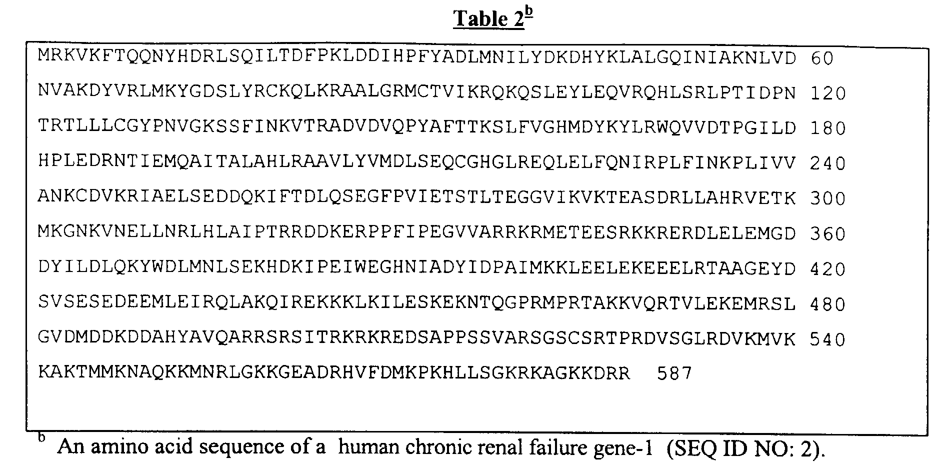

One polynucleotide of the present invention encoding chronic renal failure gene-1 may be obtained using standard cloning and screening, from a cDNA library derived from mRNA in cells of human kidney and testes using the expressed sequence tag (EST) analysis (Adams, M.D., *et al. Science* (1991) 252:1651-1656; Adams, M.D. *et al., Nature,* (1992) *355:* 632-634; Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding chronic renal failure gene-1 polypeptide of SEQ ID NO:2 may be identical to the polypeptide encoding sequence contained in Table 1 (nucleotide number 456 to 2216 of SEQ ID NO:1), or it may be a sequence, which as a result of the redundancy (degeneracy) of the genetic code, also encodes the polypeptide of SEQ ID NO:2.

When the polynucleotides of the invention are used for the recombinant production of chronic renal failure gene-1 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al., Proc Natl Acad Sci USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding chronic renal failure gene-1 variants comprising the amino acid sequence of chronic renal failure gene-1 polypeptide of Table 2 (SEQ ID NO:2) in which several, 5-10, 1-5, 1-3, 1-2 or 1 amino acid residues are substituted, deleted or added, in any combination. Among the preferred polynucleotides of the present invention is contained in Table 3 (SEQ ID NO: 3) encoding the amino acid sequence of Table 4 (SEQ ID NO: 4).

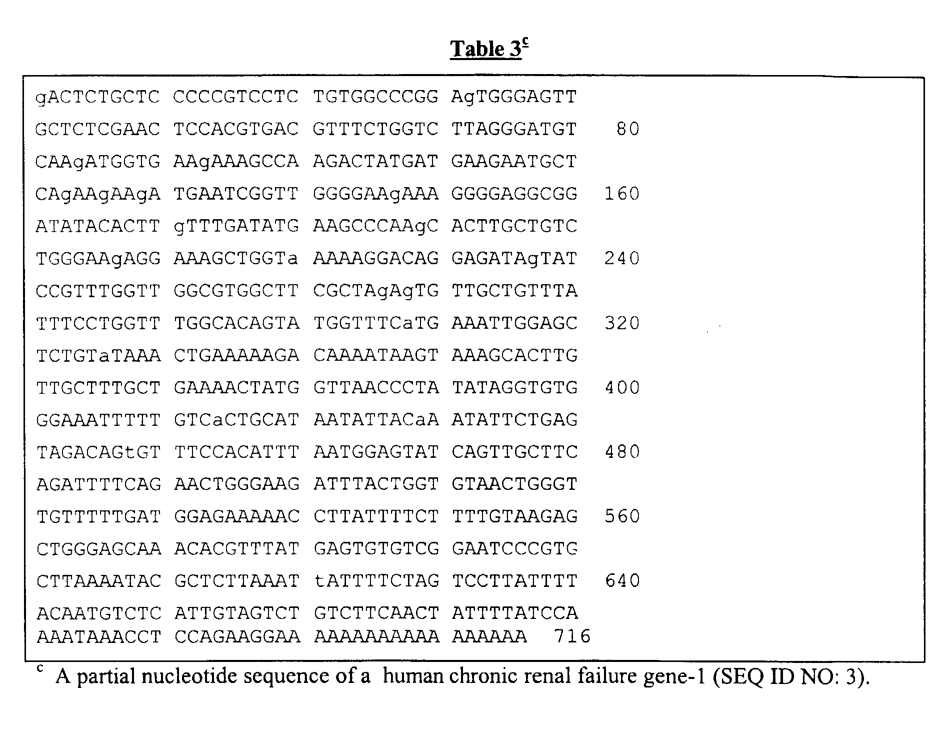

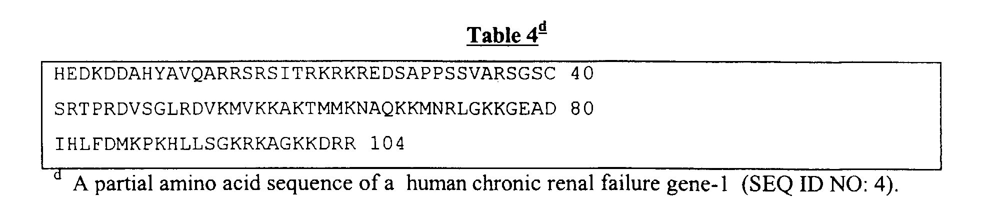

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 80%, and preferably at least 90%, and more preferably at least 95%, yet even more preferably 97-99% identity between the sequences.

Polynucleotides of the invention, which are identical or sufficiently identical to a nucleotide sequence contained in SEQ ID NO: 1 or a fragment thereof (including that of SEQ ID NO:3), may be used as hybridization probes for cDNA and genomic DNA, to isolate full-length cDNAs and genomic clones encoding chronic renal failure gene-1 polypeptide and to isolate cDNA and genomic clones of other genes (including genes encoding homologs and orthologs from species other than human) that have a high sequence similarity to the chronic renal failure gene-1 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding chronic renal failure gene-1 polypeptide, including homologs and orthologs from species other than human, comprises the steps of screening an appropriate library under stingent hybridization conditions with a labeled probe having the SEQ ID NO: 1 or a fragment thereof (including that of SEQ ID NO: 3), and isolating full-length cDNA and genomic clones containing said polynucleotide sequence. Such hybridization techniques are well known to those of skill in the art. Thus in another aspect, chronic renal failure gene-1 polynucleotides of the present invention further include a nucleotide sequence comprising a nucleotide sequence that hybridize under stringent condition to a nucleotide sequence having SEQ ID NO: 1 or a fragment thereof (including that of SEQ ID NO:3). Also included with chronic renal failure gene-1 polypeptides are polypeptide comprising amino acid sequence encoded by nucleotide sequence obtained by the above hybridization condition. Stringent hybridization conditions are as defined above or, alternatively, conditions under overnight incubation at 42°C in a solution comprising: 50% formamide, 5xSSC (150mM NaCI, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING*: *A LABORATORY MANUAL,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al., MOLECULAR CLONING, A LABORATORY MANUAL (supra).*

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the chronic renal failure gene-1 polypeptide is to be expressed for use in screening assays, generally, it is preferred that the polypeptide be produced at the surface of the cell. In this event, the cells may be harvested prior to use in the screening assay. If chronic renal failure gene-1 polypeptide is secreted into the medium, the medium can be recovered in order to recover and purify the polypeptide; if produced intracellularly, the cells must first be lysed before the polypeptide is recovered.

Chronic renal failure gene-1 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to the use of chronic renal failure gene-1 polynucleotides for use as diagnostic reagents. Detection of a mutated form of chronic renal failure gene-1 gene associated with a dysfunction will provide a diagnostic tool that can add to or define a diagnosis of a disease or susceptibility to a disease which results from under-expression, over-expression or altered expression of chronic renal failure gene-1. Individuals carrying mutations in the chronic renal failure gene-1 gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled chronic renal failure gene-1 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising chronic renal failure gene-1 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee *et al., Science,* Vol 274, pp 610-613 (1996)).

The diagnostic assays offer a process for diagnosing or determining a susceptibility to chronic renal disease, renal ischemia, diabetic nephropathy, acute renal failure, and Alzheimer's disease through detection of mutation in the chronic renal failure gene-1 gene by the methods described.

In addition, chronic renal disease, renal ischemia, diabetic nephropathy, acute renal failure, and Alzheimer's disease, can be diagnosed by methods comprising determining from a sample derived from a subject an abnormally decreased or increased level of chronic renal failure gene-1 polypeptide or chronic renal failure gene-1 mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an chronic renal failure gene-1 polypeptide, in a sample derived from a host are well-known to those of skill in the art. Such assay methods include radioimmunoassays, competitive-binding assays, Western Blot analysis and ELISA assays.

Thus in another aspect, the present invention relates to a diagonostic kit for a disease or suspectability to a disease, particularly chronic renal disease, renal ischemia, diabetic nephropathy, acute renal failure, Alzheimer's disease, which comprises:
(a) a chronic renal failure gene-1 polynucleotide, preferably the nucleotide sequence of SEQ ID NO: 1, or a fragment thereof;
(b) a nucleotide sequence complementary to that of (a);
(c) a chronic renal failure gene-1 polypeptide, preferably the polypeptide of SEQ ID NO: 2, or a fragment thereof; or
(d) an antibody to a chronic renal failure gene-1 polypeptide, preferably to the polypeptide of SEQ ID NO: 2.
It will be appreciated that in any such kit, (a), (b), (c) or (d) may comprise a substantial component.

### Chromosome Assays

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes). The differences in the cDNA or genomic sequence between affected and unaffected individuals can also be determined. If a mutation is observed in some or all of the affected individuals but not in any normal individuals, then the mutation is likely to be the causative agent of the disease.

The chronic renal failure gene-1 is believed to be on chromosome 10p15.2 - 15.3, which is associated with glioma of the brain.

### Antibodies

The polypeptides of the invention or their fragments or analogs thereof, or cells expressing them can also be used as immunogens to produce antibodies immunospecific for the chronic renal failure gene-1 polypeptides. The term "immunospecific" means that the antibodies have substantiall greater affinity for the polypeptides of the invention than their affinity for other related polypeptides in the prior art.

Antibodies generated against the chronic renal failure gene-1 polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al., Immunology Today* (1983) 4:72) and the EBV-hybridoma technique (Cole *et al.,* MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against chronic renal failure gene-1 polypeptides may also be employed to treat chronic renal disease, renal ischemia, diabetic nephropathy, acute renal failure, and Alzheimer's disease, among others.

### Vaccines

Another aspect of the invention relates to a method for inducing an immunological response in a mammal which comprises inoculating the mammal with chronic renal failure gene-1 polypeptide, or a fragment thereof, adequate to produce antibody and/or T cell immune response to protect said animal from chronic renal disease, renal ischemia, diabetic nephropathy, acute renal failure, and Alzheimer's disease, among others. Yet another aspect of the invention relates to a method of inducing immunological response in a mammal which comprises, delivering chronic renal failure gene-1 polypeptide via a vector directing expression of chronic renal failure gene-1 polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a chronic renal failure gene-1 polypeptide wherein the composition comprises a chronic renal failure gene-1 polypeptide or chronic renal failure gene-1 gene. The vaccine formulation may further comprise a suitable carrier. Since chronic renal failure gene-1 polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The chronic renal failure gene-1 polypeptide of the present invention may be employed in a screening process for compounds which activate (agonists) or inhibit activation of (antagonists, or otherwise called inhibitors) the chronic renal failure gene-1 polypeptide of the present invention. Thus, polypeptides of the invention may also be used to assess identify agonist or antagonists from, for example, cells, cell-free preparations, chemical libraries, and natural product mixtures. These agonists or antagonists may be natural or modified substrates, ligands, enzymes, receptors, etc., as the case may be, of the polypeptide of the present invention; or may be structural or functional mimetics of the polypeptide of the present invention. See Coligan *et al., Current Protocols in Immunology* 1(2):Chapter 5 (1991).

Chronic renal failure gene-1 polypeptides are responsible for many biological functions, including many pathologies. Accordingly, it is desirous to find compounds and drugs which stimulate chronic renal failure gene-1 polypeptide on the one hand and which can inhibit the function of chronic renal failure gene-1 polypeptide on the other hand. In general, agonists are employed for therapeutic and prophylactic purposes for such conditions as chronic renal disease, renal ischemia, diabetic nephropathy, acute renal failure, and Alzheimer's disease. Antagonists may be employed for a variety of therapeutic and prophylactic purposes for such conditions as chronic renal disease, renal ischemia, diabetic nephropathy, acute renal failure, and Alzheimer's disease.

In general, such screening procedures may involve using appropriate cells which express the chronic renal failure gene-1 polypeptide or respond to chronic renal failure gene-1 polypeptide of the present invention. Such cells include cells from mammals, yeast, *Drosophila* or *E. coli.* Cells which express the chronic renal failure gene-1 polypeptide (or cell membrane containing the expressed polypeptide) or respond to chronic renal failure gene-1 polypeptide are then contacted with a test compound to observe binding, or stimulation or inhibition of a functional response. The ability of the cells which were contacted with the candidate compounds is compared with the same cells which were not contacted for chronic renal failure gene-1 activity.

The assays may simply test binding of a candidate compound wherein adherence to the cells bearing the chronic renal failure gene-1 polypeptide is detected by means of a label directly or indirectly associated with the candidate compound or in an assay involving competition with a labeled competitor. Further, these assays may test whether the candidate compound results in a signal generated by activation of the chronic renal failure gene-1 polypeptide, using detection systems appropriate to the cells bearing the chronic renal failure gene-1 polypeptide. Inhibitors of activation are generally assayed in the presence of a known agonist and the effect on activation by the agonist by the presence of the candidate compound is observed.

Further, the assays may simply comprise the steps of mixing a candidate compound with a solution containing a chronic renal failure gene-1 polypeptide to form a mixture, measuring chronic renal failure gene-1 activity in the mixture, and comparing the chronic renal failure gene-1 activity of the mixture to a standard.

The chronic renal failure gene-1 cDNA, protein and antibodies to the protein may also be used to configure assays for detecting the effect of added compounds on the production of chronic renal failure gene-1 mRNA and protein in cells. For example, an ELISA may be constructed for measuring secreted or cell associated levels of chronic renal failure gene-1 protein using monoclonal and polyclonal antibodies by standard methods known in the art, and this can be used to discover agents which may inhibit or enhance the production of chronic renal failure gene-1 (also called antagonist or agonist, respectively) from suitably manipulated cells or tissues.

The chronic renal failure gene-1 protein may be used to identify membrane bound or soluble receptors, if any, through standard receptor binding techniques known in the art. These include, but are not limited to, ligand binding and crosslinking assays in which the chronic renal failure gene-1 is labeled with a radioactive isotope (eg 125I), chemically modified (eg biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. In addition to being used for purification and cloning of the receptor, these binding assays can be used to identify agonists and antagonists of chronic renal failure gene-1 which compete with the binding of chronic renal failure gene-1 to its receptors, if any. Standard methods for conducting screening assays are well understood in the art.

Examples of potential chronic renal failure gene-1 polypeptide antagonists include antibodies or, in some cases, oligonucleotides or proteins which are closely related to the ligands, substrates, enzymes, receptors, etc., as the case may be, of the chronic renal failure gene-1 polypeptide, e.g., a fragment of the ligands, substrates, enzymes, receptors, etc.; or small molecules which bind to the polypeptide of the present invention but do not elicit a response, so that the activity of the polypeptide is prevented.

Thus in another aspect, the present invention relates to a screening kit for identifying agonists, antagonists, ligands, receptors, substrates, enzymes, etc. for chronic renal failure gene-1 polypeptides; or compounds which decrease or enhance the production of chronic renal failure gene-1 polypeptides, which comprises:
(a) a chronic renal failure gene-1 polypeptide, preferably that of SEQ ID NO:2;
(b) a recombinant cell expressing a chronic renal failure gene-1 polypeptide, preferably that of SEQ ID NO:2;
(c) a cell membrane expressing a chronic renal failure gene-1 polypeptide; preferably that of SEQ ID NO: 2; or
(d) antibody to a chronic renal failure gene-1 polypeptide, preferably that of SEQ ID NO: 2. It will be appreciated that in any such kit, (a), (b), (c) or (d) may comprise a substantial component.

### Prophylactic and Therapeutic Methods

This invention provides methods of treating abnormal conditions such as, chronic renal disease, renal ischemia, diabetic nephropathy, acute renal failure, Alzheimer's disease, related to both an excess of and insufficient amounts of chronic renal failure gene-1 polypeptide activity.

If the activity of chronic renal failure gene-1 polypeptide is in excess, several approaches are available. One approach comprises administering to a subject an inhibitor compound (antagonist) as hereinabove described along with a pharmaceutically acceptable carrier in an amount effective to inhibit the function of the chronic renal failure gene-1 polypeptide, such as, for example, by blocking the binding of ligands, substrates, enzymes, receptors, etc., or by inhibiting a second signal, and thereby alleviating the abnormal condition. In another approach, soluble forms of chronic renal failure gene-1 polypeptides still capable of binding the ligand, substrate, enzymes, receptors, etc. in competition with endogenous chronic renal failure gene-1 polypeptide may be administered. Typical embodiments of such competitors comprise fragments of the chronic renal failure gene-1 polypeptide.

In another approach, soluble forms of chronic renal failure gene-1 polypeptides still capable of binding the ligand in competition with endogenous chronic renal failure gene-1 polypeptide may be administered. Typical embodiments of such competitors comprise fragments of the chronic renal failure gene-1 polypeptide.

In still another approach, expression of the gene encoding endogenous chronic renal failure gene-1 polypeptide can be inhibited using expression blocking techniques. Known such techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science* (1991) 251:1360. These oligomers can be administered *per se* or the relevant oligomers can be expressed *in vivo.*

For treating abnormal conditions related to an under-expression of chronic renal failure gene-1 and its activity, several approaches are also available. One approach comprises administering to a subject a therapeutically effective amount of a compound which activates chronic renal failure gene-1 polypeptide, i.e., an agonist as described above, in combination with a pharmaceutically acceptable camer, to thereby alleviate the abnormal condition. Alternatively, gene therapy may be employed to effect the endogenous production of chronic renal failure gene-1 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, *Gene Therapy and other Molecular Genetic-based Therapeutic Approaches,* (and references cited therein) in Human Molecular Genetics, T Strachan and A P Read, BIOS Scientific Publishers Ltd (1996). Another approach is to administer a therapeutic amount of chronic renal failure gene-1 polypeptides in combination with a suitable pharmaceutical carrier.

### Formulation and Administration

Peptides, such as the soluble form of chronic renal failure gene-1 polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo,* and for example, by the use of a retroviral plasmid vector. The cells are then introduced into the subject.

### Example 1

The existence of CRFG-1 was determined by differential display polymerase chain reaction (DDPCR) as developed by Liang P. and Pardee A.B. Science. 1992 Aug 14; Volume 257, pages 967-71. An oligonucleotide primer (Primer I), 5'-ACCACACATCTGA- 3' (SEQ ID NO:5) was used in the synthesis of complementary DNA (cDNA) from RNA of lean and obese Zucker rat kidneys. cDNA was synthesized in a 20 µl volume with 0.5 µg RNA, 1 µM primer I, 20 µM dNTP, 400 units M-MLV reverse transcriptase (Promega, Madison ,WI), and 1X standard reverse transcriptase buffer as given by the manufacturer. The reaction sample was heated to 65 degrees C for 5 minutes and then incubated at 42 degrees C for one hour. Polymerase chain reaction (PCR) was then performed in a 20 µl reaction volume using 4 µl of the cDNA synthesis reaction from above with 1.5 mM MgCl₂, 20 µM dNTP, 1 µM primer I, 1 µM primer II (5'-TGTTGGGAACAAG -3') (SEQ ID NO:6), 2 µCi [33-P]-d-alpha-ATP, 1.25 U Amplitaq polymerase (Perkin Elmer, Foster City, CA) and 1X standard PCR buffer from the manufacturer. PCR cycling conditions were 40 cycles of 94C for 30 sec, 42C for 2 min, 72C for 30 sec with a final extension of 72C for 5 min. Labeled PCR fragments from lean and obese Zucker rat Kidney RNA were resolved on a 12% SDS-polyacrylamide gel and exposed to X-ray film for 16 hours. A PCR amplified DNA fragment of 225 nucleotide size was identified to be decreased in obese Zucker rat kidneys compared to lean age matched control rats. The fragment was excised from the dried polyacrylamide gel and DNA was eluted with boiling water. The eluted DNA was subjected to PCR using the same conditions as above. A 2 µl aliquot of the PCR reaction was then used to subclone the PCR fragment into the pCRII vector (Invitrogen, Carlsbad, CA) using standard reaction conditions of the manufacturer. The cDNA insert was then sequenced with the fmol sequencing kit (Promega, Madison, WA).

The sequence information was used to generate an anti-sense primer for the cloning of additional 5' sequence using the Marathon RACE kit (Clonetech, Palo, Alto, CA). The 607 nucleotide sequence obtained from the Marathon RACE kit was then used to identify the human homologue using BLAST sequence analysis algorithm. The human sequence is given in SEQ ID NO:1.

### Example2

CRFG-1 mRNA was detected by northern blot in RNA from rat kidneys. RNA was extracted. Total RNA was extracted from renal cortex by guanidinium thiocyanate denaturation and acidified phenol-chloroform extraction (CHOMCZYNSKI P, SACCHI N: Analyt Biochem 162:156-159, 1987). Total RNA (10 µg) was fractionated on 0.2M formaldehyde-1% agarose gels and transferred to nylon membranes (Nylon-1, Gibco-BRL, Gaithersburg, MD) in 4X standard saline citrate. Equivalent loading and transfer were verified by methylene blue staining. Antisense [32P]cDNA probes were made for CRFG-1 that recognizes mRNA from rat at 1.5 and 2.5 kb. Northern blot analysis showed that CRFG-1 mRNA was decreased in kidneys from obese Zucker rats which develop renal failure. CRFG-1 mRNA was also decreased in kidneys after partial nephrectomy where 5/6 of the total renal mass was removed. This animal model develops chronic renal disease (Shea SM, Raskova J, Morrison AB Am J Pathol 1980 Aug; 100(2):513-528 ). CRFG-1 mRNA was also decreased in kidneys of aging F344 rats. F344 rats develop renal disease with advancing age (McDermott GF, Ingram A, Scholey J, Kirkland JL, Whiteside CI J Gerontol A Biol Sci Med Sci 1996 Mar; 51(2):M80-M85), suggesting that CRFG-1 is decreased in renal disease.

All publications, including but not limited to patents and patent applications, cited in this specification are herein incorporated by reference as if each individual publication were specifically and individually indicated to be incorporated by reference herein as though fully set forth.

## Claims

1. An isolated polynucleotide comprising a nucleotide sequence that has at least 80% identity over its entire length to a nucleotide sequence encoding the chronic renal failure gene-1 polypeptide of SEQ ID NO:2; or a nucleotide sequence complementary to said isolated polynucleotide.

2. The polynucleotide of claim 1 wherein said polynucleotide comprises the nucleotide sequence contained in SEQ ID NO: 1 encoding the chronic renal failure gene-1 polypeptide of SEQ ID NO2.

3. The polynucleotide of claim 1 wherein said polynucleotide comprises a nucleotide sequence that is at least 80% identical to that of SEQ ID NO: 1 over its entire length.

4. The polynucleotide of claim 3 which is the polynucleotide of SEQ ID NO: 1.

5. The polynucleotide of claim 1 which is DNA or RNA.

6. A DNA or RNA molecule comprising an expression system, wherein said expression system is capable of producing a chronic renal failure gene-1 polypeptide comprising an amino acid sequence, which has at least 80% identity with the polypeptide of SEQ ID NO:2 when said expression system is present in a compatible host cell.

7. A host cell comprising the expression system of claim 6.

8. A process for producing a chronic renal failure gene-1 polypeptide comprising culturing a host of claim 7 under conditions sufficient for the production of said polypeptide and recovering the polypeptide from the culture.

9. A process for producing a cell which produces a chronic renal failure gene-1 polypeptide thereof comprising transforming or transfecting a host cell with the expression system of claim 6 such that the host cell, under appropriate culture conditions, produces a chronic renal failure gene-1 polypeptide.

10. A chronic renal failure gene-1 polypeptide comprising an amino acid sequence which is at least 80% identical to the amino acid sequence of SEQ ID NO:2 over its entire length.

11. The polypeptide of claim 10 which comprises the amino acid sequence of SEQ ID NO:2.

12. An antibody immunospecific for the chronic renal failure gene-1 polypeptide of claim 10.

13. A method for the treatment of a subject in need of enhanced activity or expression of chronic renal failure gene-1 polypeptide of claim 10 comprising:
(a) administering to the subject a therapeutically effective amount of an agonist to said polypeptide; and/or
(b) providing to the subject an isolated polynucleotide comprising a nucleotide sequence that has at least 80% identity to a nucleotide sequence encoding the chronic renal failure gene-1 polypeptide of SEQ ID NO:2 over its entire length; or a nucleotide sequence complementary to said nucleotide sequence in a form so as to effect production of said polypeptide activity *in vivo.*

14. A method for the treatment of a subject having need to inhibit activity or expression of chronic renal failure gene-1 polypeptide of claim 10 comprising:
(a) administering to the subject a therapeutically effective amount of an antagonist to said polypeptide; and/or
(b) administering to the subject a nucleic acid molecule that inhibits the expression of the nucleotide sequence encoding said polypeptide; and/or
(c) administering to the subject a therapeutically effective amount of a polypeptide that competes with said polypeptide for its ligand, substrate , or receptor.

15. A process for diagnosing a disease or a susceptibility to a disease in a subject related to expression or activity of chronic renal failure gene-1 polypeptide of claim 10 in a subject comprising:
(a) determining the presence or absence of a mutation in the nucleotide sequence encoding said chronic renal failure gene-1 polypeptide in the genome of said subject; and/or
(b) analyzing for the presence or amount of the chronic renal failure gene-1 polypeptide expression in a sample derived from said subject.

16. A method for identifying compounds which inhibit (antagonize) or agonize the chronic renal failure gene-1 polypeptide of claim 10 which comprises:
(a) contacting a candidate compound with cells which express the chronic renal failure gene-1 polypeptide (or cell membrane expressing chronic renal failure gene-1 polypeptide) or respond to chronic renal failure gene-1 polypeptide; and
(b) observing the binding, or stimulation or inhibition of a functional response; or comparing the ability of the cells (or cell membrane) which were contacted with the candidate compounds with the same cells which were not contacted for chronic renal failure gene-1 polypeptide activity.

17. An agonist identified by the method of claim 16.

18. An antagonist identified by the method of claim 16.

19. A recombinant host cell produced by a method of Claim 9 or a membrane thereof expressing a chronic renal failure gene-1 polypeptide.
